Europäisches Patentamt

(19) European Patent Office

Office européen des brevets

(11) EP 0 609 217 B1

(12) EUROPEAN PATENT SPECIFICATION

(45) Date of publication and mention
of the grant of the patent:
18.02.1998 Bulletin 1998/08

(51) Int. Cl.⁶: A61K 33/18, A61K 47/48,
A01N 59/12, A61K 31/715

(21) Application number: 92906739.5

(22) Date of filing: 18.03.1992

(86) International application number:
PCT/CA92/00115

(87) International publication number:
WO 92/17190 (15.10.1992 Gazette 1992/26)

(54) **DRY STARCH-IODINE PHARMACEUTICAL FORMULATIONS**

TROCKENE STÄRKE-IOD ENTHALTENDE PHARMAZEUTISCHE FORMULIERUNGEN

PREPARATIONS PHARMACEUTIQUES SECHES A BASE D'AMIDON/IODE

(84) Designated Contracting States:
AT BE CH DE DK ES FR GB GR IT LI LU MC NL
SE

(30) Priority: 28.03.1991 US 676170

(43) Date of publication of application:
10.08.1994 Bulletin 1994/32

(73) Proprietors:
• 943038 Ontario Inc.
Kingston, Ontario K7L 3G4 (CA)
• Eskin, Bernard A., Dr.
Bala Cynwyd, Pennsylvania 19004 (US)

(72) Inventors:
• Ghent, William R., Dr.
Kingston, Ontario K7L 3G6 (CA)
• Eskin, Bernard A., Dr.
Bala Cynwyd, Pa. 19004 (US)

(74) Representative:
Griffin, Kenneth David et al
Saunders & Dolleymore,
9, Rickmansworth Road
Watford, Hertfordshire WD1 7HE (GB)

(56) References cited:
EP-A- 0 006 340          EP-A- 0 124 774
EP-A- 0 377 266          WO-A-85/02422
CH-A-   124 440          US-A- 2 022 729
US-A- 2 927 058          US-A- 4 010 259

• WORLD PATENTS INDEX Section Ch, Week
6800, Derwent Publications Ltd., London, GB;
Class B, AN 66-32598F & SU,A,197 385
(MOKHNACH VO)
• Lexicon der Hilfstoffe, 1989, page 152
• Organic Chemistry, Kemp and Vellaccio, page
992-4
• Hatch Anal. Chem, 1982, 54; 2002-5

Remarks:
The file contains technical information submitted
after the application was filed and not included in
this specification

## Description

### BACKGROUND OF THE INVENTION

The present invention is concerned with the use of starch as a complexant with iodine for preparing dry powder pharmaceutical formulations useful in the preparation of capsules or tablets.

More particularly, the invention is concerned with the administration of molecular iodine ($I_2$) to patients suffering from iodine deficiency diseases by administering to said patients a starch iodine complex in a dry formulation.

Heretofore, iodine deficiency diseases have been treated by administering to patients, in need thereof, an aqueous solution containing elemental iodine ($I_2$) (U.S. Patent No. 4,816,255 to Ghent et al. issued March 28, 1989 and WO90/07339, Ghent et al. published July 12, 1990).

The administration of aqueous molecular iodine to patients has several disadvantages including the need for specialized dispensers utilizing selective membranes thereby preventing the patient from ingesting crystallized iodine. Standardization of daily dosage is also a problem associated with these dispensers. See U.S. Patent No. 4,384,960 to Polley. In WO90/07339, Ghent et al. published July 12, 1990, a method was described for providing an aqueous solution of elemental iodine free of any micro or macro particles of iodine. In this method iodine crystals were placed in a sealed plastic bag or container which was exposed to water at about 20°C. The iodine crystals sublime, and iodine vapour passes through the plastic and into the water to produce a pure solution of elemental iodine without any particulate matter. The temperature of the water effects the rate of sublimation of the prilled iodine, and therefore effects the time of stabilization of the pure solution at the required concentration. Therefore standardization of daily dose is also a problem with this prior art method. Furthermore, this prior art method of preparing the pure solution of elemental iodine is not a convenient method for use by the general public.

In view of the problems associated with the prior art methods, a therapeutic effective amount of elemental iodine in a convenient form and standardized dose has been desired.

### SUMMARY STATEMENT OF THE INVENTION

The present invention therefore relates to the preparation of a dry form of iodine, which may be encapsulated in capsule or pill form, for therapeutic oral administration to patients.

More particularly, the invention relates to the use of starch, and more particularly to the use of starch containing a high proportion of amylose, as a complexant with iodine to provide means to prepare a dry powder formulation in capsule or tablet form for oral administration to patients suffering from or for prevention of various iodine deficiency diseases and the like.

The present invention also relates to the manufacture of a dry pharmaceutical composition useful in the treatment of iodine deficiency diseases including breast dysplasia, breast cancer, endometriosis and premenstrual syndrome as well as other iodine related diseases, said composition comprising the dry starch-iodine complex and, if desired, in combination with one or more suitable adjuvants. The present invention also relates to the treatement of iodine deficieny diseases by administering an effective amount of the dry pharmaceutical compositon.

### BRIEF DESCRIPTION OF THE DRAWING

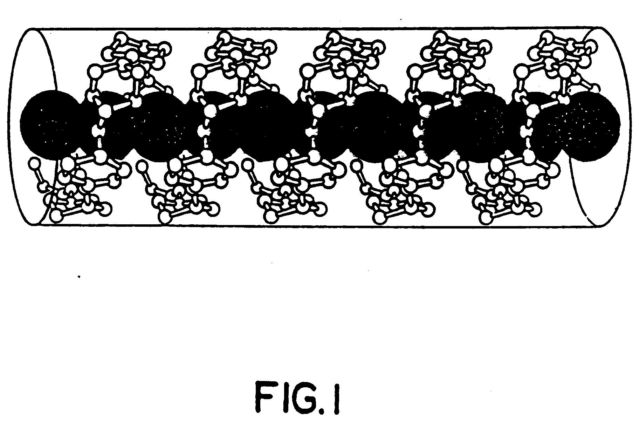

Fig. 1 is a molecular representation of the amylose-iodine complex wherein the iodine molecules depicted as solid spheres fit within the cavity of the amylose helices.

### DESCRIPTION OF THE PREFERRED EMBODIMENTS

In view of the apparent disadvantages to the administration of aqueous molecular iodine, it became evident that it would be desirable to administer the iodine in capsule or tablet form. The present invention is directed to dry pharmaceutical formulations containing iodine and the administration of the same in capsule or tablet form. This mode of administration of iodine is believed to be superior to those prior art methods and overcomes the various disadvantages experienced by the administration of elemental iodine in aqueous form.

Starch, in general, contains a mixture of a water-soluble straight-chain fraction called amylose, and a water-insoluble branched fraction called amylopectin. Depending upon the source of the starch, these two fractions appear in different proportions. Upon treatment of acid or under the influence of enzymes, the components of starch are hydrolysed progressively to dextrin, which is a mixture of low-molecular-weight polysaccharides, (+)-maltose, and finally D-(+)-glucose. Both amylose and amylopectin are made up of D-(+)-glucose units, but differ in molecular size and shape.

Of particular interest, to the present invention, is the structure of amylose which is a linear 1,4-acetal polymer of glucose. Thus, amylose is made up of chains of many D-(+)-glucose units, each C-1 of each glucose unit joined by an $\alpha$-glycoside linkage to the C-4 of the next glucose unit. It is the alpha linkage at the acetal carbon which has a profound effect on the overall shape of the giant amylose molecule which gives it its unique physical properties and the interactions the amylose molecule may have with smaller molecules.

Amylose is a helical molecule, with glucose residues that coil back on each other, creating a loosely overlapping spiral with a central cavity or tube. A variety of small molecules including triiodide ions ($I_3^-$) or polyiodide ions ($I_5^-$) up to ($I_{11}^-$) form stable complexes with amylose. As is known, the starch-iodine complex has a deep blue-violet colour that can be used to test for the presence of either amylose or iodine. Furthermore, the decolorization of an amylose/iodine complex has been used as a test for amylase activity for some time. From structural evidence, it is apparent that the colour arises because of interactions between rows of $I_3^-$ molecules oriented end to end inside the tubular cavity of amylose structure (see Organic Chemistry, Kemp-Vellaccio, Worth Publishers, Inc., copyright 1980, p. 994).

It is the unique physical properties of the amylose molecule which forms a part of starch which has led us to believe that starch would be ideally suited as a complexant for iodine to prepare dry pharmaceutical formulations which may be encapsulated in capsule or tablet form for oral administration. These dry formulations of iodine would be superior to known aqueous iodine solution used in the treatment of iodine deficiency diseases such as breast dysplasia, breast cancer, endometriosis and premenstrual syndrome. Such aqueous formulations, containing elemental iodine ($I_2$), are contemplated in U.S. Patent No. 4,816,255 to Ghent et al issued March 28, 1989, and WO90/07339, published July 12, 1990. Dry powder formulations of the present form in capsule or tablet form are also useful in the treatment of radiation sickness from nuclear fallout and the like and are more easily administrable in oral form to patients.

The present invention is concerned with utilizing starch as a complexant with iodine to form an iodine-starch complex and more specifically with the use of the amylose component of starch to form an amylose-iodine complex as depicted in Fig. 1. (Taken from Organic Chemistry, Kemp-Vellaccio, Worth Publishers, Inc. 1980, p. 994.)

It is mostly the linear component of starch which is responsible for the uptake of iodine. This starch-iodine complex exists in a helical configuration as depicted in Fig. 1 within which the triiodide ions ($I_3^-$) or polyiodide ions ($I_5^-$ up to $I_{11}^-$) reside. The most common complex however is the triiodide ion-amylose complex. The molecular weight or molecular weight distribution of the starch (amylose) plays a critical factor in the ability of starch to complex iodine.

The minimum length of the amylose chain to produce its characteristic blue colour is about 30-40 D-glucose units, providing a cavity corresponding closely in length to an 11-atom polyiodide chain. The brand, lot, quality, source, time of harvesting and place of growth are all factors which will influence the linear amylose content of the starches. In the present invention, starch containing a high proportion of amylose is preferred. As such the amount of iodine in the complex is determined by the amount of iodine supplied to the complex rather that the amount of iodine that can be bound by the starch amylose. A preferred starch for use in the preparation of the starch-iodine formulation is #7 Hylon starch. This starch is a purified "specialty" starch produce by Nacan Products of Toronto, Ontario, Canada, a subsidiary of National Starch & Chemical Company of New Jersey. The starch is made from a hybrid corn called Hylon #7 and contains 70 percent amylose and 30 percent amylopectin.

In the preparation of the iodine-starch (amylose) complex, since the iodine will ultimately be used to treat human patients, high quality re-sublimed iodine should be employed in the preparation.

In determining a method for the production of the desired iodine-starch complex, the effect of temperature on the capacity of starch to absorb iodine must be examined. The effective temperature is best described by Hatch (Analytical Chemistry, vol. 54, 2002 (1982). At higher temperature (especially at 60°C), there is a temperature-dependant change in the structure and (or) number of starch helices available for reaction with iodine (triiodide ions). In fact, the thermal decoloration of the blue starch-iodine complex is the result of thermally induced deterioration or unravelling of the starch helices from around the iodine (triiodide ions ($I_3^-$) or others). This phenomenon is called hysteresis effect and is reversible at least up to 40°C. It has been found with the starch-iodine complex of the present invention that the molecule will begin to unravel at 40°C. Colorimetric changes begin to occur at about 50°C to 60°C, with complete discolorization occurring at about 60°C to about 80°C.

As noted above, the iodine found in the starch-iodine complex is primarily in the form of triiodide ions ($I_3^-$). However, as noted in the prior art, the iodine used for the treatment or prevention of iodine deficiency diseases is elemental or molecular iodine, $I_2$. Therefore, the present invention is primarily concerned with the preparation of a dry formulation of iodine for oral consumption, which will supply elemental iodine ($I_2$) for treatment of iodine deficiency diseases and the like. It was unexpectedly found that a dry starch-iodine complex containing primarily $I_3^-$ would upon the administration to patients provide the required $I_2$ for the necessary treatment or prevention of iodine deficiency diseases.

In order to understand how $I_2$ is supplied to the patient, one must understand how iodine in predominately triiodide form is released from starch. There are three different methods for releasing the iodine from the starch molecule which include:

a) thermally with temperatures greater than 40°C, which induces the unfolding of the amylose helices;
b) by acid hydrolysis of starch down to D-glucose in aqueous solutions; or
c) by $\alpha$-amylase treatment.

For a detailed description of the last two methods see, for example, Robyt, Starch, Chemistry and Technology, p. 94.

It is the latter treatment, i.e. that of $\alpha$-amylase which

is involved in the in vivo degradation of starch. Salivary α-amylase degrades starch (amylose) to higher oligosaccharides and ultimately to glucose. In the stomach, the further α-amylase activity causes further starch degradation and liberation of iodine ($I_3^-$). The other enzymes which further degrade these fragments are glucosidases (α-1→4 and α-1→6). These enzymes are secreted by the brush border cells of the lining of the small intestine and by the pancreas. These enzymes do not have any action on starch per se, but their combined action completes the job of converting starch into D-glucose.

In one embodiment of the present invention, the starch-iodine complex is encapsulated, for example with gelatin. The encapsulated starch-iodine complex reaches the stomach intact. The stomach acids soften the gelatin capsule so that its contents, i.e., the starch-iodine complex, is released in the upper small bowel. The complex is then acted upon by the upper small bowel contents including α-amylase, bile and pancreatic enzymes containing amylase and glucosidases. The amylase, aided by bile, will digest the amylose and thereby release the $I_3^-$ from the complex.

$I_3^-$ cannot exist as a molecule (uncomplexed) and immediately changes to $I_2$ and $I^-$. The $I^-$ is picked up by the sodium, potassium and proteins of the food stuffs to produce iodides, while the $I_2$ is absorbed as $I_2$ apparently in the same fashion as $I_2$ administered in an aqueous vehicle.

It can be seen from the foregoing, that the starch-iodine ($I_3^-$) complex was found, according to the present invention, to be ideally suited to produce a dry formulation for oral administration of iodine due to the immediate conversion of the triiodide ion to $I_2$ in vivo.

The starch-iodine complex of the present invention, in capsule form, has been used as a replacement to treat women who were formally treated for iodine deficiency disorders with the aqueous formulation. The results obtained with the dry formulation are the same as those already reported for the aqueous formulation.

While this invention is described in detail with particular reference to certain preferred embodiments thereof, the following examples are offered to illustrate but not limit the invention.

## EXAMPLE 1

The starch-iodine complex of the present invention can generally be prepared by the following method. In this method, the preferred starch, #7 Hylon is used. This specialized starch is exposed to aqueous molecular iodine (AMI) at room temperature (20°C) (300 milligrams iodine per liter) in a ratio of 1 gram of starch to 100 milliliters AMI for 12 hours at 20°C.

The iodine solution is prepared by exposing 50 grams of prilled iodine to ion-free water at 20°C for 4 days.

The chemical reaction of this combination is

$$I_2 + H_2O = HOI + I^- + I_2 + I_3^-.$$

The proportion of $I_2$ and $I_3^-$ are temperature dependant as noted above, with an increased temperature increasing the amount of $I_3^-$.

The solution is passed through a micropore filter and is then ready for combining with the starch. The AMI solution combines with the amylose of starch and produces a complex of primarily $I_3^-$ with the amylose helices. The $I_2$ and $I^-$ present in the AMI solution are compressed in the helices to $I_3^-$. It is this combination that produces the colorimetric change to blue.

After exposure of the AMI to starch for 12 hours, the supernatant water is decanted and tested for presence of iodine.

The resulting starch-iodine complex is vacuum dried at room temperature and then mechanically pulverized. It is now ready for encapsulation, preferably at a strength of 3 milligrams of iodine in 100 milligrams of the complex.

The above dosage of 3 milligrams of iodine in 100 milligrams of the complex is the preferred daily dosage for the treatment of iodine deficiency diseases such as breast dysplasia, breast cancer, endometriosis and premenstrual syndrome as disclosed WO90/07339 to Ghent et al, published July 12, 1990. A daily dosage of up to about .08 milligrams per kilogram body weight is appropriate.

The ideal complex would contain sufficient amount of iodine in the starch-iodine complex to yield 3 milligrams of $I_2$ in 100 milligrams starch so that direct encapsulation without fillers could be used. However, the amount of iodine/amylose configuration could be changed if encapsulation required it.

It is to be understood that the recommended daily dosage of $I_2$ for the treatment of other iodine related diseases such as with the thyroid including radiation sickness etc. may be other than the preferred dosage disclosed hereinabove and thus variation of the amount of aqueous molecular iodine supplied to starch may be increased or decreased depending on the dosage required. Specialty starches such as #7 Hylon starch with its high amylose content are uniquely suited for complexing of an increased amount of iodine and as indicated previously the amount of iodine in the complex is a function of the amount of iodine supplied to the complex rather than the amount of iodine that can be bound by the starch amylose. Other less amylose rich starches may be used however provided there is sufficient amylose present to supply the desired dosage of iodine.

Although the use of additional pharmaceutical adjuvants is believed not to be required, suitable pharmaceutical adjuvants, fillers, excipients etc. may form part of the pharmaceutical composition if desired.

## EXAMPLE 2

A dry starch-iodine complex suitable for therapeutic use can also be made by a allowing the vapour of prilled iodine to come in contact with the starch.

The preferred starch, Hylon #7, (100 grams) was exposed to 20 grams of prilled iodine in such a fashion that there is no direct contact between the two components. The iodine sublimes and complexes with the starch due to the minute amount of moisture in the starch.

The process is carried out in a sealed container and the contact between the starch and iodine vapour is maintained for 10 days at 20°C. The end point is colorimetrically judged at 3 milligrams of iodine complexed with 100 milligrams of starch.

## Claims

**Claims for the following Contracting States : AT, BE, CH, DE, DK, FR, GB, IT, LI, LU, MC, NL, SE**

1. The use of an effective amount of a dry starch-iodine complex for the preparation of a medicament in capsule, tablet or pill form which when ingested in vivo delivers an effective amount of $I_2$ for the treatment of iodine deficiency diseases selected from the group consisting of breast dysplasia, breast cancer, endometriosis, premenstrual syndrome and radiation sickness.

2. The use of Claim 1, wherein the starch-iodine complex contains a sufficient amount of iodine to yield about 3 milligrams of $I_2$ per 100 milligrams of starch.

3. The use of Claim 1, wherein the starch-iodine complex contains $I_3^-$, and $I_5^-$ up to $I_{11}^-$.

4. A method of preparing a dry starch-iodine complex as defined in Claim 1 for the preparation of capsules, pills or tablets for oral administration to patients suffering from iodine deficiency diseases comprising preparing an aqueous solution of iodine, wherein the iodine is substantially elemental iodine ($I_2$) and exposing starch (amylose) to the iodine solution for a sufficient time to allow the iodine to complex with the starch and drying the starch-iodine complex.

5. The method of Claim 4, wherein the dry starch-iodine complex is encapsulated into capsule, tablet or pill form.

6. The method of Claim 4, wherein the iodine solution is prepared by exposing 50g of prilled iodine to ion-free water at room temperature for 4 days, said solution then being passed through a micropore filter.

7. The method of Claim 6, wherein starch having a suitable amylose component is exposed to said aqueous molecular iodine at room temperature for sufficient period of time to allow complexing to occur.

8. The method according to any one of Claims 4 to 7, wherein the starch-iodine complex is dried at room temperature followed by mechanical pulverization thereof thereby producing a dry powder starch-iodine complex.

9. The method of Claim 8, further comprising mixing the dry powder starch-iodine complex with suitable therapeutically acceptable adjuvants prior to encapsulation.

**Claims for the following Contracting States : ES, GR**

1. A method of preparing a dry starch-iodine complex suitable for the preparation of capsules, pills or tablets for oral administration to patients suffering from iodine deficiency diseases comprising preparing an aqueous solution of iodine, wherein the iodine is substantially elemental iodine ($I_2$) and exposing starch (amylose) to the iodine solution for a sufficient time to allow the iodine to complex with the starch and drying the starch-iodine complex.

2. The method of Claim 1 wherein the dry starch-iodine complex is encapsulated into capsule, tablet or pill form.

3. The method of Claim 1 wherein the starch-iodine complex is used in the prevention of radiation sickness.

4. The method of Claim 1 wherein the iodine solution is prepared by exposing 50g of prilled iodine to ion-free water at room temperature for 4 days, said solution then being passed through a micropore filter.

5. The method of Claim 4 wherein starch having a suitable amylose component is exposed to said aqueous molecular iodine at room temperature for sufficient period of time to allow complexing to occur.

6. The method according to any one of Claims 1 to 5 wherein the starch-iodine complex is dried at room temperature followed by mechanical pulverisation thereof thereby producing a dry powder starch-iodine complex.

7. The method of Claim 1 wherein the iodine in the dry starch-iodine complex is in the form of $I_3^-$, and $I_5^-$ up to $I_{11}^-$.

8. The method of Claim 1 wherein the starch contains a high proportion of amylose.

9. A method for preparing a pharmaceutical composition useful in the treatment of iodine deficiency diseases including breast dysplasia, breast cancer, endometriosis and premenstrual syndrome comprising encapsulating a dry starch-iodine complex into capsule, tablet or pill form.

10. The use of an effective amount of a dry starch-iodine complex for the preparation of a medicament in capsule, tablet or pill form which when ingested in vivo delivers an effective amount of $I_2$ for the treatment of iodine deficiency diseases selected from the group consisting of breast dysplasia, breast cancer, endometriosis, premenstrual syndrome and radiation sickness.

11. The use of Claim 10, wherein the starch-iodine complex contains a sufficient amount of iodine to yield about 3 milligrams of $I_2$ per 100 milligrams of starch.

12. The use of Claim 10, wherein the starch-iodine complex contains $I_3^-$, and $I_5^-$ up to $I_{11}^-$.

**Patentansprüche**

**Patentansprüche für folgende Vertragsstaaten : AT, BE, CH, DE, DK, FR, GB, IT, LI, LU, MC, NL, SE**

1. Verwendung einer wirksamen Menge eines trockenen Stärke-Iod-Komplexes zur Herstellung eines Arzneimittels in Form von Kapseln, Tabletten oder Pillen, welches bei der Einnahme in vivo eine wirksame Menge $I_2$ zuführt zur Behandlung von Iodmangelerkrankungen, die aus der Gruppe Dysplasie der Brust, Brustkrebs, Endometriose, prämenstruelles Syndrom und Strahlenkrankheit ausgewählt sind.

2. Verwendung nach Anspruch 1, wobei der Stärke-Iod-Komplex eine solche Menge an Iod enthält, die ausreicht, um etwa 3 mg $I_2$ pro 100 mg Stärke zu ergeben.

3. Verwendung nach Anspruch 1, wobei der Stärke-Iod-Komplex $I_3^-$ und $I_5^-$ bis zu $I_{11}^-$ enthält.

4. Verfahren zur Herstellung eines trockenen Stärke-Iod-Komplexes gemäß der Definition in Anspruch 1 zur Herstellung von Kapseln, Pillen oder Tabletten zur oralen Verabreichung an Patienten, die an Iodmangelerkrankungen leiden, welches die Herstellung einer wäßrigen Lösung von Iod, in der Iod im wesentlichen als elementares Iod ($I_2$) vorliegt, und Kontaktierung von Stärke (Amylose) mit der Iodlösung während einer solchen Dauer, die zur Komplexbildung des Iods mit der Stärke ausreicht, und Trocknen des Stärke-Iod-Komplexes, umfaßt.

5. Verfahren nach Anspruch 4, wobei der trockene Stärke-Iod-Komplex in einer Kapsel, Tablette oder in Pillenform eingekapselt wird.

6. Verfahren nach Anspruch 4, wobei die Iodlösung hergestellt wird, indem 50 g granuliertes Iod bei Raumtemperatur 4 Tage lang in entionisiertem Wasser gehalten wird und die Lösung danach durch ein Mikroporenfilter geleitet wird.

7. Verfahren nach Anspruch 6, wobei Stärke, die einen geeigneten Amylose-Bestandteil enthält, bei Raumtemperatur während einer solchen Dauer mit der wäßrigen Lösung von molekularem Iod in Kontakt gehalten wird, die ausreicht, daß Komplexbildung eintritt.

8. Verfahren nach einem der Ansprüche 4 bis 7, wobei der Stärke-Iod-Komplex bei Raumtemperatur getrocknet und danach unter Bildung eines trockenen pulverförmigen Stärke-Iod-Komplexes mechanisch pulverisiert wird.

9. Verfahren nach Anspruch 8, welches weiterhin das Vermischen des trockenen pulverförmigen Stärke-Iod-Komplexes vor dem Einkapseln mit passenden therapeutisch geeigneten Zusätzen umfaßt.

**Patentansprüche für folgende Vertragsstaaten : ES, GR**

1. Verfahren zur Herstellung eines trockenen Stärke-Iod-Komplexes, der zur Herstellung von Kapseln, Pillen oder Tabletten zur oralen Verabreichung an Patienten, die an Iodmangelerkrankungen leiden, geeignet ist, welches die Herstellung einer wäßrigen Lösung von Iod, in der Iod im wesentlichen als elementares Iod ($I_2$) vorliegt, und Kontaktierung von Stärke (Amylose) mit der Iodlösung während einer solchen Dauer, die zur Komplexbildung des Iods mit der Stärke ausreicht, und Trocknen des Stärke-Iod-Komplexes, umfaßt.

2. Verfahren nach Anspruch 1, wobei der trockene Stärke-Iod-Komplex in einer Kapsel, Tablette oder in Pillenform eingekapselt wird.

3. Verfahren nach Anspruch 1, wobei der Stärke-Iod-Komplex zum Verhüten von Strahlenkrankheit verwendet wird.

4. Verfahren nach Anspruch 1, wobei die Iodlösung hergestellt wird, indem 50 g granuliertes Iod bei Raumtemperatur 4 Tage lang in entionisiertem Wasser gehalten wird und die Lösung danach durch ein Mikroporenfilter geleitet wird.

5. Verfahren nach Anspruch 4, wobei Stärke, die einen geeigneten Amylose-Bestandteil enthält, bei Raumtemperatur während einer solchen Dauer mit der wäßrigen Lösung von molekularem Iod in Kontakt gehalten wird, die ausreicht, daß Komplexbildung eintritt.

6. Verfahren nach einem der Ansprüche 1 bis 5, wobei der Stärke-Iod-Komplex bei Raumtemperatur getrocknet und danach unter Bildung eines trockenen pulverförmigen Stärke-Iod-Komplexes mechanisch pulverisiert wird.

7. Verfahren nach Anspruch 1, wobei das Iod in dem trockenen Stärke-Iod-Komplex in Form von $I_3^-$ und $I_5^-$ bis zu $I_{11}^-$ vorliegt.

8. Verfahren nach Anspruch 1, wobei die Stärke einen hohen Anteil an Amylose enthält.

9. Verfahren zur Herstellung einer Arzneimittelzusammensetzung, die sich zur Behandlung von Iodmangelerkrankungen einschließlich Dysplasie der Brust, Brustkrebs, Endometriose und prämenstruelles Syndrom eignet, welches das Einkapseln eines trockenen Stärke-Iod-Komplexes in die Form von Kapseln, Tabletten oder Pillen umfaßt.

10. Verwendung einer wirksamen Menge eines trockenen Stärke-Iod-Komplexes zur Herstellung eines Arzneimittels in Form von Kapseln, Tabletten oder Pillen, welches bei der Einnahme in vivo eine wirksame Menge $I_2$ zuführt, zur Behandlung von Iodmangelerkrankungen, die aus der Gruppe Dysplasie der Brust, Brustkrebs, Endometriose, prämenstruelles Syndrom und Strahlenkrankheit ausgewählt sind.

11. Verwendung nach Anspruch 10, wobei der Stärke-Iod-Komplex eine solche Menge an Iod enthält, die ausreicht um etwa 3 mg $I_2$ pro 100 mg Stärke zu ergeben.

12. Verwendung nach Anspruch 10, wobei der Stärke-Iod-Komplex $I_3^-$ und $I_5^-$ bis $I_{11}^-$ enthält.

**Revendications**

**Revendications pour les Etats contractants suivants : AT, BE, CH, DE, DK, FR, GB, IT, LI, LU, MC, NL, SE**

1. Utilisation d'une quantité efficace d'un complexe sec d'amidon-iode pour la préparation d'un médicament sous la forme de gélules, comprimés ou pilules qui, lorsqu'il est ingéré in vivo, libère une quantité efficace de $I_2$ pour le traitement des maladies dues à une carence en iode, choisies parmi le groupe constitué des dysplasie du sein, cancer du sein, endométriose, syndrome prémenstruel et maladies dues aux radiations.

2. Utilisation selon la revendication 1, dans laquelle le complexe d'amidon-iode contient une quantité suffisante d'iode pour produire environ 3 milligrammes de $I_2$ pour 100 milligrammes d'amidon.

3. Utilisation selon la revendication 1, dans laquelle le complexe d'amidon-iode contient de l'iode sous forme de $I_3^-$, et $I_5^-$ jusqu'à $I_{11}^-$.

4. Procédé de préparation d'un complexe sec d'amidon-iode selon la revendication 1, adapté à la préparation de gélules, pilules ou comprimés destinés à une administration orale aux patients qui souffrent de maladies dues à une carence en iode, comprenant la préparation d'une solution aqueuse d'iode, dans laquelle l'iode est sensiblement de l'iode élémentaire ($I_2$), puis l'exposition d'amidon (amylose) à cette solution d'iode pendant un temps suffisamment long pour permettre à l'iode de former un complexe avec l'amidon, et enfin le séchage du complexe amidon-iode.

5. Procédé selon la revendication 4, dans lequel le complexe sec d'amidon-iode est encapsulé sous forme de gélule, pilule ou comprime.

6. Procédé selon la revendication 4, dans lequel la solution d'iode est préparée en exposant 50 g d'iode en granulés à de l'eau déminéralisée, à la température ambiante, pendant 4 jours, ladite solution étant ensuite filtrée à travers un filtre microporeux.

7. Procédé selon la revendication 6, dans lequel l'amidon ayant un composant amylose approprié est exposé à cet iode moléculaire aqueux, à la température ambiante, pendant une période suffisamment longue pour permettre la formation du complexe.

8. Procédé selon l'une quelconque des revendications 4 à 7, dans lequel le complexe d'amidon-iode est séché à la température ambiante, puis est soumis à une pulvérisation mécanique, ce qui produit un complexe pulvérulent sec d'amidon-iode.

9. Procédé selon la revendication 8, comprenant en outre le mélange du complexe pulvérulent sec d'amidon-iode avec des adjuvants appropriés au plan thérapeutique, avant l'encapsulage.

**Revendications pour les Etats contractants suivants : ES, GR**

1. Procédé de préparation d'un complexe sec d'amidon-iode adapté à la préparation de gélules, pilules ou comprimés destinés à une administration orale aux patients qui souffrent de maladies dues à une carence en iode, comprenant la préparation d'une solution aqueuse d'iode, dans laquelle l'iode est sensiblement de l'iode élémentaire ($I_2$), puis l'exposition d'amidon (amylose) à cette solution d'iode pendant un temps suffisamment long pour permettre à l'iode de former un complexe avec l'amidon, et enfin le séchage du complexe amidon-iode.

2. Procédé selon la revendication 1, dans lequel le complexe sec d'amidon-iode est encapsulé sous forme de gélule, pilule ou comprimé.

3. Procédé selon la revendication 1, dans lequel le complexe d'amidon-iode est utilisé dans la prévention des maladies dues aux radiations.

4. Procédé selon la revendication 1, dans lequel la solution d'iode est préparée en exposant 50 g d'iode en granulés à de l'eau déminéralisée, à la température ambiante, pendant 4 jours, ladite solution étant ensuite filtrée à travers un filtre microporeux.

5. Procédé selon la revendication 4, dans lequel l'amidon ayant un composant amylose approprié est exposé à cet iode moléculaire aqueux, à la température ambiante, pendant une période suffisamment longue pour permettre la formation du complexe.

6. Procédé selon l'une quelconque des revendications 1 à 5, dans lequel le complexe d'amidon-iode est séché à la température ambiante, puis est soumis à une pulvérisation mécanique, ce qui produit un complexe pulvérulent sec d'amidon-iode.

7. Procédé selon la revendication 1, dans lequel l'iode du complexe sec d'amidon-iode se présente sous la forme $I_3^-$, et $I_5^-$ jusqu'à $I_{11}^-$.

8. Procédé selon la revendication 1, dans lequel l'amidon contient une forte proportion d'amylose.

9. Procédé de préparation d'une composition pharmaceutique utile pour le traitement des maladies dues à une carence en iode, y compris la dysplasie du sein, le cancer du sein, l'endométriose et le syndrome prémenstruel, comprenant l'encapsulage d'un complexe sec d'amidon-iode sous la forme de gélules, comprimés ou pilules.

10. Utilisation d'une quantité efficace d'un complexe sec d'amidon-iode pour la préparation d'un médicament en gélules, comprimés ou pilules qui, lorsqu'il est ingéré in vivo, libère une quantité efficace de $I_2$ pour le traitement des maladies dues à une carence en iode, choisies parmi le groupe constitué des dysplasie du sein, cancer du sein, endométriose, syndrome prémenstruel et maladies dues aux radiations.

11. Utilisation selon la revendication 10, dans laquelle le complexe d'amidon-iode contient une quantité suffisante d'iode pour produire environ 3 milligrammes de $I_2$ pour 100 milligrammes d'amidon.

12. Utilisation selon la revendication 10, dans laquelle le complexe d'amidon-iode contient de l'iode sous forme de $I_3^-$, et $I_5^-$ jusqu'à $I_{11}^-$.

# FIG.I